# EUROPÄISCHE PATENTSCHRIFT

(11) **EP 4 147 678 B1**
(45) Veröffentlichungstag und Bekanntmachung des Hinweises auf die Patenterteilung: **08.11.2023**
(21) Anmeldenummer: 21195922.6
(22) Anmeldetag: 10.09.2021
(51) Int. Cl.: A61F 2/30, A61F 2/46

(54) **AUGMENTATIONSVORRICHTUNG UND AUGMENTATIONSSYSTEM**
AUGMENTATION DEVICE AND AUGMENTATION SYSTEM
DISPOSITIF D'AUGMENTATION ET SYSTÈME D'AUGMENTATION

(43) Veröffentlichungstag der Anmeldung: 15.03.2023
(73) Patentinhaber: Heraeus Medical GmbH, 61273 Wehrheim (DE)
(72) Erfinder: VOGT, Sebastian, 63450 Hanau (DE); KLUGE, Thomas, 63450 Hanau (DE)
(74) Vertreter: Heraeus IP

(56) Entgegenhaltungen:
- US-A1- 2011 009 974
- US-A1- 2018 008 416
- US-A1- 2018 200 061
- US-A1- 2018 271 658

## Beschreibung

Die Erfindung ist durch die unabhängigen Ansprüche 1 und 11 definiert.

Sie betrifft eine Augmentationsvorrichtung umfassend einen ringförmigen Konus, der einen Kanal umgibt, welcher sich von einem proximalen Konusende zu einem distalen Konusende des Konus durch den Konus erstreckt.

Die Erfindung betrifft weiterhin ein Augmentationssystem umfassend eine derartige Augmentationsvorrichtung und einen Applikator.

Gegenstand der Erfindung ist insbesondere eine Augmentationsvorrichtung zum Einsatz bei Gelenkendoprothesenoperationen, insbesondere Revisionsgelenkendoprothesenoperationen.

Die erfindungsgemäße Augmentationsvorrichtung ist dazu geeignet, debridiertes Knochengewebe zu verstärken oder gar teilweise zu ersetzen und so eine sichere und stabile Verankerung einer Gelenkendoprothese, insbesondere einer Revisionsendoprothese, in einem Knochenkanal zu ermöglichen. Zudem dient die erfindungsgemäße Augmentationsvorrichtung insbesondere einer gleichmäßigen Krafteinleitung in das umgebende Knochengewebe im Zuge einer Implantation einer Gelenkendoprothese, insbesondere einer Revisionsgelenkendoprothese

### Hintergrund der Erfindung

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Dabei kann es im Bereich der entfernten Gelenkendoprothese zu einem substanziellen Verlust an Knochengewebe, insbesondere bei vorgeschädigter Knochengewebesubstanz, beispielsweise durch Osteoporose, kommen, was zu Problemen bei der Verankerung einer Revisionsgelenkendoprothese führen kann. Um das verbliebene Knochengewebe zu verstärken oder teilweise gar zu ersetzen und eine möglichst gleichmäßige Krafteinleitung in das verbliebene Knochengewebe beim Einsatz der Revisionsgelenkendoprothese zu gewährleisten, besteht eine mögliche Behandlungsoption in einem Einsatz einer Augmentationsvorrichtung in Form eines Metallkonus. Dazu wird das infizierte Knochengewebe abgetragen und die dadurch entstandene Kavität durch Einsetzten der Augmentationsvorrichtung aufgefüllt. Die konusförmige Ausgestaltung der Augmentationsvorrichtung dient dazu, einen Schaft einer Gelenkendoprothese, beziehungsweise einer Revisionsgelenkendoprothese, in einen zentral verlaufenden Kanal der Augmentationsvorrichtung aufzunehmen und dort, beispielsweise unter Einsatz von Knochenzement, zu verankern. Die Augmentationsvorrichtung selbst ist so gestaltet, dass sie in die durch das Debridement entstandene Kavität eingebracht werden kann.

Eine derartige Augmentationsvorrichtung ist beispielsweise in der Patentschrift US 8,506,645 B2 beschrieben.

Üblicherweise weisen die im Markt erhältlichen Augmentationsvorrichtungen eine definierte, vorbestimmte Größe auf und können nicht an die jeweilige anatomische Gegebenheit des Patienten angepasst werden. Daher werden im Markt Augmentationsvorrichtungen unterschiedlicher Größe angeboten.

In der Patentschrift EP 2 319 558 B1 wird eine Augmentationsvorrichtung beschrieben, deren Größe, insbesondere deren Außendurchmesser, in bestimmten Grenzen variiert werden kann. Dazu weist die Augmentationsvorrichtung einen ringförmigen Konus auf, welcher zumindest ein axial verlaufendes Biegegelenk aufweist. Durch das zumindest eine Gelenk lässt sich der Konus durch einen von außen einwirkenden Druck zusammendrücken, was den Außendurchmesser der Augmentationsvorrichtung verringert.

Als nachteilig ist bei einer Verwendung von Gelenken, insbesondere Biegegelenken anzusehen, dass die Anpassung der Größe, insbesondere des Außendurchmessers, der Augmentationsvorrichtung mit einer deutlichen Verformung der Augmentationsvorrichtung einhergeht. Die Augmentationsvorrichtung weist durch die Anpassung der Größe einen "Knick" auf, welcher die Einpassung der Augmentationsvorrichtung in den Knochen des Patienten erschwert und sich nachteilig bei der Implantation der Gelenkendoprothese, beziehungsweise der Revisionsgelenkendoprothese, auswirken kann. Zudem ist eine Anpassung der Größe der Augmentationsvorrichtung nur in einem geringen Umfang möglich. Eine Anpassung der axialen Erstreckung der Augmentationsvorrichtung ist nicht möglich.

Eine weitere konusförmige Augmentationsvorrichtung ist in der US 2018/271658 A1 offenbart. Durch Schneiden kann der Anwender die Größe der Augmentationsvorrichtung an den Patienten anpassen. Diese Anpassung ist folglich irreversibel. US 2018/271658 A1 offenbart die technischen Merkmale der Präambel des Anspruchs 1.

Wünschenswert ist daher eine Augmentationsvorrichtung, welche einfach und schnell in ihrer Größe anpassbar ist. Die Anpassung der Größe soll ohne eine Verformung der Augmentationsvorrichtung möglich sein. Weiterhin soll die Anpassung der Größe ohne Einsatz von Werkzeugen manuell innerhalb weniger Sekunden im Zuge einer Operation möglich sein.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll eine Augmentationsvorrichtung bereitgestellt werden, welche einfach und schnell in ihrer Größe an die anatomischen Gegebenheiten eines Patienten angepasst werden kann. Weiterhin soll die Augmentationsvorrichtung bei der Anpassung der Größe nicht verformt werden.

Es ist eine weitere Aufgabe der Erfindung ein Augmentationssystem zur einfachen und schnellen Implantation einer Augmentationsvorrichtung in einen Patienten zur Verfügung zu stellen, welches Beschränkungen herkömmlicher Augmentationssysteme vermeidet.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Augmentationsvorrichtung umfassend einen ringförmigen Konus, der einen Kanal umgibt, welcher sich von einem proximalen Konusende zu einem distalen Konusende des Konus durch den Konus erstreckt, wobei der Konus aus einer Vielzahl miteinander verbundener, ringförmiger Konussegmente ausgebildet ist, wobei ein Konussegmentaußendurchmesser der einzelnen Konussegmente vom proximalen Konusende zum distalen Konusende abnimmt und wobei die Konussegmente trennbar, insbesondere zerstörungsfrei trennbar, voneinander ausgestaltet sind, so dass der Konus eine adaptierbare Konusgröße aufweist.

Die Konussegmente sind formschlüssig, kraftschlüssig oder formschlüssig und kraftschlüssig miteinander verbunden.

In einer Ausführungsform der Augmentationsvorrichtung sind die Konussegmente reversibel trennbar voneinander ausgestaltet. Diese Ausführungsform ist eine zweite Ausführungsform der Erfindung, welche vorzugsweise von der ersten Ausführungsform der Erfindung abhängt.

In einer Ausführungsform der Augmentationsvorrichtung sind die Konussegmente mittels einer Nut-Feder-Verbindung miteinander verbunden. Diese Ausführungsform ist eine dritte Ausführungsform der Erfindung.

In einer Ausführungsform der Augmentationsvorrichtung weisen die Konussegmente jeweils eine Ausnehmung, einen Steg oder eine Ausnehmung und einen Steg auf, wobei die Ausnehmung und der Steg zweier im Konus benachbarter Konussegmente zusammenwirken, um die zwei im Konus benachbarten Konussegmente miteinander zu verbinden, insbesondere reversibel miteinander zu verbinden. Diese Ausführungsform ist eine vierte Ausführungsform der Erfindung.

In einer Ausführungsform der Augmentationsvorrichtung sind beziehungsweise ist der Steg und/oder die Ausnehmung an einer Konussegmentinnenfläche angeordnet. Diese Ausführungsform ist eine fünfte Ausführungsform der Erfindung.

In einer Ausführungsform der Augmentationsvorrichtung weist der Konus eine dem Kanal abgewandte, im Wesentlichen stufenfreie Konusaußenfläche auf. Diese Ausführungsform ist eine sechste Ausführungsform der Erfindung.

In einer Ausführungsform der Augmentationsvorrichtung weisen die Konussegmente jeweils mindestens eine Führung auf, um den Konus mit einem Applikator reversibel zu verbinden. Diese Ausführungsform ist eine siebte Ausführungsform der Erfindung.

In einer Ausführungsform der Augmentationsvorrichtung ist die Führung dem Kanal zugewandt, so dass der Applikator durch Einbringen in den Kanal mit dem Konus reversibel verbindbar ist. Diese Ausführungsform ist eine achte Ausführungsform der Erfindung.

In einer Ausführungsform der Augmentationsvorrichtung sind die Konussegmente jeweils in Form eines geschlossenen Rings ausgebildet. Diese Ausführungsform ist eine neunte Ausführungsform der Erfindung.

In einer Ausführungsform der Augmentationsvorrichtung umfassen die Konussegmente ein Polymer, insbesondere einem Polymethylmethacrylat, ein Metall, insbesondere Titan oder Tantal, eine Metalllegierung, insbesondere eine Titanlegierung, eine Tantallegierung oder einen Edelstahl, oder eine Kombination der vorgenannten. Insbesondere bestehen die Konussegmente aus einem Polymer, insbesondere einem Polymethylmethacrylat, einem Metall, insbesondere Titan oder Tantal, oder einer Metalllegierung, insbesondere einer Titanlegierung, einer Tantallegierung oder einem Edelstahl. Diese Ausführungsform ist eine zehnte Ausführungsform der Erfindung.

Eine elte Ausführungsform der Erfindung ist ein Augmentationssystem umfassend eine Augmentationsvorrichtung nach einer der vorhergehenden Ausführungsformen und einen Applikator der Applikator umfassend einen Griff und ein mit dem Griff verbundenes Halteelement, wobei das Halteelement derart ausgeformt ist, dass bei einem Einschieben des Applikators in den Kanal das Haltelement an einer Konusinnenfläche anliegt und so die Augmentationsvorrichtung und den Applikator reversibel miteinander verbindet.

In einer Ausführungsform des Augmentationssystems ist der Applikator in den Kanal vom proximalen Konusende aus einschiebbar. Diese Ausführungsform ist eine zwölfte Ausführungsform der Erfindung.

In einer Ausführungsform des Augmentationssystems sind an dem Halteelement Halteelementeinkerbungenen, insbesondere treppenartig ausgeformte Halteelementeinkerbungen, ausgebildet, welche mit Konuseinkerbungen, insbesondere mit treppenartig ausgeformten Konuseinkerbunden, an der Konusinnenfläche in Eingriff gebracht werden können. Diese Ausführungsform ist eine dreizehnte Ausführungsform der Erfindung.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich entgegengesetzten Enden der Augmentationsvorrichtung, des Konus, oder anderer Struktureinheiten der Augmentationsvorrichtung und des Augmentationssystems und erlauben keinen Rückschluss auf die Orientierung der in einen menschlichen Körper implantieren Augmentationsvorrichtung. "Distal zu ..." und "proximal zu..." oder ähnliche Formulierungen drücken entsprechend lediglich die räumliche Anordnung zweier Struktureinheiten der Augmentationsvorrichtung und des Augmentationssystems zueinander aus.

### Ausführliche Beschreibung

Ein erster Gegenstand der Erfindung betrifft eine Augmentationsvorrichtung umfassend einen ringförmigen Konus, der einen Kanal umgibt, welcher sich von einem proximalen Konusende zu einem distalen Konusende des Konus durch den Konus erstreckt, wobei der Konus aus einer Vielzahl miteinander verbundener, ringförmiger Konussegmente ausgebildet ist, wobei ein Konussegmentaußendurchmesser der einzelnen Konussegmente vom proximalen Konusende zum distalen Konusende abnimmt und wobei die Konussegmente trennbar voneinander ausgestaltet sind, so dass der Konus eine adaptierbare Konusgröße aufweist.

Die Augmentationsvorrichtung weist einen ringförmigen Konus auf. Ein Konus ist ein kegelartiges, insbesondere kegelstumpfartig, geformtes Bauteil mit einem proximalen Konusende und einem dem proximalen Konusende axial gegenüberliegendem distalen Konusende, wobei sich ein Konusaußendurchmesser von einem Konusende zum anderen Konusende, hier vom proximalen Konusende zum distalen Konusende, hin verringert.

Der Konus ist ringförmig, oder in anderen Worten rohrartig, ausgestaltet. Dadurch umschließt der Konus einen Kanal, welcher sich axial durch den Konus vom proximalen Ende bis zum distalen Ende erstreckt. Der Kanal wird durch eine Konusinnenfläche ausgebildet. Der Begriff "ringförmig" umfasst in einer axialen Aufsicht auf die Konussegmente Körper mit einem kreisförmigen, elliptischen, eckigen, beispielweise vier-, fünf- oder sechseckigen, und unregelmäßigen Umfang, wobei Körper mit elliptischem Umfang aufgrund einer besseren Implantierbarkeit in einen Patienten, insbesondere in einen Knochenkanal eines Patienten, bevorzugt sind.

Der Kanal dient der Aufnahme und Fixierung eines Schafts einer Gelenkendoprothese oder einer Revisionsgelenkendoprothese. Dafür kann der Schaft der entsprechenden Endoprothese in den Kanal, insbesondere vom proximalen Kanalende aus, eingebracht werden. Der Kanal weist einen Kanaldurchmesser auf, welcher von einem Konusinnendurchmesser bestimmt ist. In einer Ausgestaltungsform kann der Konusinnendurchmesser über die gesamte axiale Erstreckung des Kanals beziehungsweise des Konus konstant ausgestaltet sein. In einer weiteren Ausgestaltungsform verringert sich der Konusinnendurchmesser gleichlaufend zum Konusaußendurchmesser vom proximalen Konusende bis zum distalen Konusende. In dieser Ausgestaltungsform weist der Konus eine über die gesamte axiale Erstreckung des Konus im Wesentlichen gleich starke Konuswanddicke auf.

Die Konuswanddicke kann beispielsweise in einem Bereich von 2 bis 30 mm, bevorzugt 2 bis 15 mm, weiter bevorzugt 2 bis 10 mm, liegen.

Der Konus wird durch eine Vielzahl, also durch zwei oder mehr, miteinander verbundener, ringförmiger Konussegmente ausgebildet. Der Konus kann beispielsweise aus 2 bis 20 Konussegmenten, bevorzugt aus 4 bis 15, weiter bevorzugt aus 4 bis 10 Konussegmenten ausgebildet sein.

Jedes Konussegment bildet dabei einen ringförmigen Abschnitt des Konus und einen entsprechenden scheibenförmigen Abschnitt des Kanals des Konus ab. Dabei sind die Konussegmente so "übereinandergestapelt", dass diese den Konus ausbilden.

Die Konussegmente weisen einen Konussegmentaußendurchmesser auf, wobei sich die jeweiligen Konussegmentaußendurchmesser der einzelnen Konussegmente unterscheiden. Das Konussegment mit dem größten Konussegmentaußendurchmesser formt das proximale Konusende aus, wobei sich der Konussegmentaußendurchmesser der sich in distaler Richtung anschließender Konussegmente immer weiter verringert, bis schließlich das Konussegment mit dem kleinsten Konussegmentaußendurchmesser das distale Konusende bildet.

Die Konussegmente können dabei Konussegmentaußendurchmesser in einem Bereich von 20 bis 60 mm, bevorzugt 25 bis 55 mm, weiter bevorzugt 30 bis 55 mm aufweisen.

Unter dem Konussegmentaußendurchmesser ist bei Konussegmenten, welche in einer axialen Aufsicht einen elliptischen Umfang aufweisen, die Länge der Hauptachse, also die längere der durch den Mittelpunkt verlaufenden Achsen gemeint.

Dies erlaubt eine gute Anpassung der Konusgröße an die anatomischen Gegebenheiten eines Patienten.

Die Konussegmente können unterschiedliche Bauhöhen umfassen. Beispielsweise besitzen die einzelnen Konussegmente eine Bauhöhe im Bereich von 1 mm bis 10 mm, bevorzugt im Bereich von 2 mm bis 8 mm. Dies erlaubt eine schnelle, effektive und gleichzeitig ausreichend kleinstufige Anpassung der axialen Erstreckung der Augmentationsvorrichtung.

In einer Ausgestaltungsform kann der Konussegmentaußendurchmesser über die gesamte axiale Erstreckung des jeweiligen Konussegments konstant sein.

In einer weiteren, bevorzugten.

Ausgestaltungsform nimmt der Konussegmentaußendurchmesser des jeweiligen Konussegments von einem proximalen Ende zu einem distalen Ende des Konussegments linear ab, so dass die miteinander verbundenen Konussegmente einen Konus mit einer im Wesentlichen stufenfreien, d.h. im Wesentlichen glatten Konusaußenfläche bilden.

In einer Ausgestaltungsform können die Konussegmente an einer dem Kanal beziehungsweise Kanalabschnitt abgewandten Konussegmentaußenfläche aufgeraut oder mit einer offenporigen Struktur ausgebildet sein, um spongiöses Knochengewebe nachzubilden.

Die einzelnen Konussegmente sind trennbar voneinander, insbesondere zerstörungsfrei trennbar voneinander, ausgestaltet, so dass die Konusgröße, insbesondere der maximale und/oder der minimale Konusaußendurchmesser und die axiale Erstreckung des Konus variierbar ist. Beispielsweise kann das Konussegment mit dem größten Konussegmentaußendurchmesser von den restlichen Konussegmenten abgetrennt werden, was den maximalen Konusaußendurchmesser auf den Konussegmentaußendurchmesser des Konussegments mit dem zweitgrößten Konussegmentaußendurchmesser verringert und gleichzeitig die axiale Erstreckung des Konus um die Bauhöhe des entfernten Konussegments verkürzt. Weitere Anpassungen der Konusgröße sind durch weiteres Entfernen zusätzlicher Konussegmente am proximalen und/oder distalen Konusende möglich, bis der Konus letztendlich nur noch aus einem Konussegmente besteht.

Das Abtrennen einzelner Konussegmente erlaubt ein einfaches und schnelles Variieren der Konusgröße und damit der Größe der Augmentationsvorrichtung auch im Laufe einer Operation. Dabei kann die Konusgröße über einen weiten Bereich der anatomischen Gegebenheiten eines Patienten angepasst werden. Gleichzeitig wird die ursprüngliche Form des Konus, bis auf den Konusaußendurchmesser und die axiale Erstreckung des Konus, beibehalten. Insbesondere hat die Anpassung der Konusgröße keinen negativen Einfluss auf die Form des Kanals.

Die Konussegmente können auf unterschiedliche Weise miteinander verbunden sein, um den Konus auszubilden. In einer Ausführungsform sind die Konussegmente nach einer Trennung nicht mehr ohne Hilfsmittel, wie beispielsweise einen Kleber oder eine Klammer, miteinander verbindbar.

Eine Ausführungsform der Augmentationsvorrichtung ist dadurch gekennzeichnet, dass die Konussegmente reversibel trennbar voneinander ausgestaltet sind. Die Konussegmente sind also nach einer Trennung wieder miteinander verbindbar. Dies erlaubt eine flexiblere Anpassung und ein "Herantasten" an die benötigte Konusgröße, ohne dass nach einer initial falschen Anpassung der Konusgröße die Augmentationsvorrichtung unbrauchbar geworden ist und entsorgt werden muss.

Die Konussegmente sind formschlüssig, kraftschlüssig oder formschlüssig und kraftschlüssig miteinander verbunden.

Dies erlaubt eine einfache, schnelle und reversible Trennung der einzelnen Konussegmente durch Auseinanderziehen oder Auseinanderdrehen. Zudem ist ein Trennen der einzelnen Konussegmente manuell, also ohne Hilfsmittel, wie beispielsweise eine Schere oder Zange, möglich.

Eine Ausführungsform der Augmentationsvorrichtung ist dadurch gekennzeichnet, dass die Konussegmente mittels einer Nut-Feder-Verbindung miteinander verbunden sein können.

Eine Nut-Feder-Verbindung verbindet die einzelnen Konussegmente form- und/oder kraftschlüssig miteinander.

Eine Ausführungsform der Augmentationsvorrichtung ist dadurch gekennzeichnet, dass die Konussegmente jeweils eine Ausnehmung, einen Steg oder eine Ausnehmung und einen Steg aufweisen können, wobei die Ausnehmung eines Konussegments mit dem Steg eines zu diesem benachbarten Konussegments derart zusammenwirken, dass diese zwei Konussegmente miteinander verbunden sind. Dabei bilden Ausnehmung und Steg eine formschlüssige, eine kraftschlüssige oder eine form- und kraftschlüssige Verbindung. Ein Steg ist eine Erhebung auf einer proximalen Konussegmentseite und/oder einer distalen Konussegmentseite eines Konussegments. Eine Ausnehmung ist eine Vertiefung auf einer proximalen und/oder distalen Konussegmentseite eines Konussegments. Steg und Ausnehmung benachbarter Konussegmente sind so aufeinander abgestimmt, dass der Steg in der Ausnehmung aufgenommen werden kann. Dabei kann der Steg die Ausnehmung vollständig ausfüllen. In einer Ausführungsform erstrecken sich der Steg und/oder die Ausnehmung kreisartig über die proximale und/oder distale Konussegmentseite des jeweiligen Konussegments. Dabei umfasst der Steg beziehungsweise die Ausnehmung den Kanal beziehungsweise den Kanalabschnitt manschettenartig. In einer weiteren Ausführungsform erstrecken sich der Steg und/oder die Ausnehmung nur abschnittsweise über die proximale und/oder distale Konussegmentseite des jeweiligen Konussegments. Dabei kann der Steg beispielsweise noppenartig ausgeformt sein, so dass die Konussegmente klemmbausteinartig miteinander verbindbar sind.

In einer Ausgestaltungsform kann es für das Konussegment mit dem größten Konussegmentaußendurchmesser ausreichend sein, einen Steg oder eine Ausnehmung auf der distalen Konussegmentseite aufzuweisen. Ebenso kann es für das Konussegment mit dem kleinsten Konussegmentaußendurchmesser ausreichend sein, einen Steg oder eine Ausnehmung auf der proximalen Konussegmentseite aufzuweisen. Die dazwischenliegenden Konussegmente können sowohl auf der proximalen als auch auf der distalen

Konussegmentseite einen Steg oder eine Ausnehmung aufweisen. Dabei ist es bevorzugt, dass die Stege und die Ausnehmungen der einzelnen Konussegmente jeweils auf der gleichen Konussegmentseite, beispielsweise alle Stege auf der distalen Konussegmentseite und alle Ausnehmungen auf der proximalen Konussegmentseite, ausgeformt sind.

Der Steg, die Ausnehmung oder, falls vorhanden, der Steg und die Ausnehmung können an unterschiedlichen Bereichen auf der proximalen, der distalen oder, falls vorhanden, auf der proximalen und der distalen Konussegmentseite angeordnet sein, solange die Stege und Ausnehmung benachbarter Konussegmente zusammenwirken können, um die Konussegmente miteinander zu verbinden.

Eine Ausführungsform der Augmentationsvorrichtung ist dadurch gekennzeichnet, dass die Ausnehmung, der Steg oder die Ausnehmung und der Steg an einer dem Kanal beziehungsweise Kanalabschnitt zugewandten Konussegmentinnenfläche angeordnet sein kann beziehungsweise sein können.

Dies bedeutet, dass Steg beziehungsweise Ausnehmung an der Grenze zwischen Konussegmentseite und Konussegmentinnenfläche ausgebildet sind. Dies erlaubt eine einfache Montage der Augmentationsvorrichtung, insbesondere auch um die Augmentationsvorrichtung nach vorangegangener Trennung zweier Konussegmente wieder zu vergrößern.

Eine Ausführungsform der Augmentationsvorrichtung ist dadurch gekennzeichnet, dass die dem Kanal abgewandte Konusaußenfläche im Wesentlichen stufenfrei sein kann.

Der Konus weist damit einen Konusaußendurchmesser auf, welcher linear vom proximalen Konusende zum distalen Konusende abnimmt. Dies erlaubt eine bestmögliche Anpassung der Konusgröße an die anatomischen Gegebenheiten eines Patienten.

Die folgenden Verfahrensschritte sind nicht Teil der vorliegenden Erfindung sondern dienen lediglich zur Veranschaulichung.

Die Augmentationsvorrichtung kann auf unterschiedliche Weise in einen Patienten implantiert werden. Beispielsweise kann die Augmentationsvorrichtung mit bloßen Händen in eine dafür vorgesehene Kavität eingebracht, insbesondere eingepresst, werden.

Um ein Einbringen an die gewünschte Position innerhalb eines Patienten zu erleichtern, ist es bevorzugt, dass die Augmentationsvorrichtung mit Hilfe eines Applikators in den Patienten implantierbar ist. Ein Applikator ist eine Vorrichtung, welche reversibel mit der Augmentationsvorrichtung verbindbar ist, so dass diese sicher und einfach an die gewünschte Stelle innerhalb des Patienten eingebracht werden kann. Nach dem Einbringen lässt sich der Applikator einfach und schnell wieder von der Augmentationsvorrichtung lösen. Ein Applikator kann beispielsweise ein Greifwerkzeug sein, mit welchem die Augmentationsvorrichtung gegriffen wird und in eine vorbereitete Kavität im Patienten eingeschoben wird.

Um das temporäre, reversible Verbinden von Augmentationsvorrichtung und Applikator zu erleichtern, ist eine Ausführungsform der Augmentationsvorrichtung dadurch gekennzeichnet, dass die einzelnen Konussegmente jeweils eine Führung aufweisen können.

Die Führungen dienen als Befestigungstelle für den Applikator an der Augmentationsvorrichtung. Beispielsweise kann der Applikator zumindest abschnittsweise in die Führung eingeschoben werden, so dass Führung und Applikator form- und/oder kraftschlüssig miteinander verbunden sind. Die einzelnen Führungen der Konussegmente können dabei derart zueinander ausgerichtet sein, dass eine zusammenhängende Konusführung ausgeformt wird, in welche der Applikator einbringbar ist.

Die Führungen der Konussegmente können an der Konussegmentaußenfläche oder der dem Kanal zugewandten Konussegmentinnenfläche angeordnet sein.

Eine Ausführungsform der Augmentationsvorrichtung ist dadurch gekennzeichnet, dass die Führung dem Kanal zugewandt, also an der Konussegmentinnenfläche angeordnet, sein kann. Der Applikator lässt sich somit durch Einbringen in den Kanal, insbesondere durch Einbringen in den Kanal aus Richtung des proximalen Konusende, reversibel mit der Augmentationsvorrichtung verbinden. Gleichzeitig bleibt dabei die Konusaußenfläche vom Applikator unbedeckt, was ein Einbringen der Augmentationsvorrichtung in einen Patienten erleichtert.

In einer Ausführungsform der Augmentationsvorrichtung können die Konussegmente einen Spalt aufweisen, so dass die Konussegmente, und damit der Konus, gemäß den Abmessungen der Spalte zusammendrückbar ist. In dieser Ausführungsform können die Konussegmente die Form eines offenen Rings aufweisen.

Dies erlaubt eine Feinjustierung der Konusgröße, insbesondere des Konusaußendurchmessers, ohne zusätzliches Entfernen oder Hinzufügen eines Konussegments am proximalen und/oder distalen Konusende. Der Spalt eines Konussegments kann dabei beispielsweise 1 bis 10 mm breit sein. Der Spalt kann durch eine den Spalt überdeckende Zunge am entsprechenden Konussegment geschützt sein.

Eine Ausführungsform der Augmentationsvorrichtung ist dadurch gekennzeichnet, dass die Konussegmente jeweils in Form eines geschlossenen Rings, also ohne Spalt, ausgebildet sein können.

Derartige Konussegmente weisen eine hohe strukturelle Integrität aus, was eine gleichmäßige Krafteintragung im Zuge einer Implantation einer Gelenkendoprothese beziehungsweise einer Revisionsgelenkendoprothese befördert.

Die Konussegmente können unterschiedliche Materialien umfassen oder aus unterschiedlichen Materialien bestehen.

Eine Ausführungsform der Augmentationsvorrichtung ist dadurch gekennzeichnet, dass die Konussegmente ein Polymer, ein Metall, eine Metalllegierung oder eine Kombination der vorgenannten umfassen können, insbesondere dass die Konussegmente aus einem Polymer, einem Metall, einer Metalllegierung oder einer Kombination der vorgenannten bestehen. Beispiele für Polymere sind Polymethylmethacrylat (PMMA) und Polycarbonat, wobei Polymethylmethacrylat (PMMA) bevorzugt ist. Beispiele für Metalle sind Titan, Tantal, Aluminium und Eisen. Beispiele für Metalllegierungen sind Titanlegierungen, insbesondere TiAl₆V₄, Tantallegierungen und Edelstähle, insbesondere Edelstahl 1.4404.

Um die Augmentationsvorrichtung besser im Patienten zu verankern kann es vorteilhaft sein, dass die Konusaußenfläche aufgeraut oder mit einer offenporigen Struktur ausgebildet ist. Falls der Konus aus einem Polymer ausgebildet ist, ist es bevorzugt, dass die aufgeraute oder offenporige Struktur mit Tantal, Titan, einer Tantallegierung oder einer Titanlegierung beschichtet ist, um ein Einwachsen von Knochengewebe in den Konus zu begünstigen.

Ein weiterer Gegenstand der Erfindung betrifft ein Augmentationssystem umfassend eine Augmentationsvorrichtung nach einer der vorhergehenden Ausführungsformen und einen Applikator. Der Applikator ist, wie obenstehend beschrieben, eine Vorrichtung, welche temporär mit der Augmentationsvorrichtung verbindbar ist, so dass diese sicher und einfach an die gewünschte Stelle innerhalb des Patienten eingebracht werden kann. Nach dem Einbringen lässt sich der Applikator einfach und schnell wieder von der Augmentationsvorrichtung lösen. Ein Applikator kann beispielsweise ein Greifwerkzeug sein, mit welchem die Augmentationsvorrichtung gegriffen wird und in eine vorbereitete Kavität im Patienten eingeschoben wird.

Um den Applikator zu bedienen, umfasst dieser einen Griff. Der Griff kann beispielsweise in Form einer Stange ausgeformt sein, welche von einem Anwender des Applikators mit einer Hand umfasst werden kann. Der Griff dient einer guten Kraftübertragung vom Anwender über den Applikator auf die Augmentationsvorrichtung im Zuge einer Implantation.

Der Applikator weist ein mit dem Griff verbundenes Halteelement auf, um den Applikator mit der Augmentationsvorrichtung reversibel zu verbinden. Das Halteelement ist so ausgeformt und dimensioniert, dass es beim Einschieben in den Kanal der Augmentationsvorrichtung an der Konusinnenfläche anliegt.

In einer Ausgestaltungsform kann das Halteelement an zumindest zwei sich

gegenüberliegenden Stellen der Konusinnenfläche anliegen, um den Applikator mit der Augmentationsvorrichtung reversibel zu verbinden. In einer weiteren Ausgestaltungsform liegt das Halteelement an mehr als zwei, insbesondere an vier unterschiedlichen Stellen der Konusinnenfläche an. Dabei ist es bevorzugt, dass die mehr als zwei, insbesondere vier Stellen möglichst äquidistant über die Konusinnenfläche verteilt sind. Dies erlaubt eine möglichst gleichmäße Kraftübertragung von Applikator auf Augmentationsvorrichtung beim Implantieren der Augmentationsvorrichtung in einen Patienten.

Um eine Kraftübertragung von Applikator auf Augmentationsvorrichtung möglichst gleichmäßig auf alle Konussegmente zu übertragen, ist es bevorzugt, dass das Halteelement eine axiale Erstreckung aufweist, welche zumindest der axialen Erstreckung des Konus entspricht. Dabei ist es bevorzugt, dass das Haltelement mit allen Konussegmenten in Kontakt steht.

Die Augmentationsvorrichtung kann eine der vorbeschriebenen Führungen an einer, einigen oder allen Konussegmenten an der Konusinnenfläche auf. Dies erleichtert das reversible Verbinden von Augmentationsvorrichtung und Applikator. Beispielsweise ist das Halteelement des Applikators stangenartig und die Führung nutartig ausgeformt, so dass das Halteelement in die Führung einschiebbar ist und Augmentationsvorrichtung und Applikator form- und/oder kraftschlüssig miteinander verbindbar sind.

Der Applikator kann dabei unterschiedlich in den Kanal der Augmentationsvorrichtung eingeführt werden.

Eine Ausführungsform des Augmentationssystems ist dadurch gekennzeichnet, dass der Applikator in den Kanal vom proximalen Konusende aus einschiebbar sein kann.

Das dem proximalen Konusende zugewandte Kanalende kann breiter als das entgegengesetzte, dem distalen Konusende zugewandte Kanalende, ausgeformt sein, was die Konstruktionsweise des Applikators, insbesondere des Halteelements vereinfacht und den Einsatz eines robusteren Applikators, beispielsweise ohne bewegliche Teile, erlaubt. Zudem kann so ein Zugang des Applikators über die gesamte axiale Erstreckung des Konus gewährleistet werden.

Das Halteelement kann unterschiedliche Geometrien aufweisen, um den Applikator mit der Augmentationsvorrichtung, insbesondere mit der Augmentationsvorrichtung an der Konusinnenfläche, reversibel zu verbinden. Beispielsweise kann das Haltelement mit einer glatten Oberfläche an die Augmentationsvorrichtung anliegen. Dabei ist es bevorzugt, dass das Halteelement und die Konusinnenfläche im Wesentlichen eine gleiche Steigung aufweisen, damit beide Bauteile über eine möglichst weite Strecke miteinander in Kontakt stehen. Eine weitere Möglichkeit besteht darin, dass Konusinnenfläche und Halteelement über ineinandergreifende Profile miteinander reversibel verbindbar sind. Die vorbeschriebenen Führungen können als Teil eines derartigen konusseitigen Profils ausgestaltet sein oder die vorbeschriebenen Führungen sind ein derartiges konusseitiges Profils.

Eine Ausführungsform des Augmentationssystems ist dadurch gekennzeichnet, dass an dem Halteelement, insbesondere an einem der Augmentationsvorrichtung zugewandten Teil des Halteelements, Halteelementeinkerbungen, insbesondere treppenartige Haltelementeinkerbungen, ausgebildet sein können, welche mit Konuseinkerbungen, insbesondere mit treppenartigen Konuseinkerbungen, an der Konusinnenfläche in Eingriff gebracht werden können. Dabei wirken die Haltelementeinkerbungen und die Konuseinkerbungen formschlüssig zusammen, so dass bei einer Kraftausübung vom Applikator auf die Augmentationsvorrichtung beim Implantieren der Augmentationsvorrichtung in einen Patienten die Gefahr eines Abrutschens des Applikators von der Konusinnenfläche verringert wird. Das Zusammenwirken von Halteelementeinkerbungen und Konuseinkerbungen verbessert die reversible Verbindung von Applikator und Augmentationsvorrichtung beim Implantieren in einen Patienten.

Dabei können die Konuseinkerbungen als Teil einer der vorbeschriebenen Führungen ausgeformt sein oder die Konuseinkerbungen stellen eine vorbeschriebene Führung dar.

Das Halteelement kann an einer dem Griff entgegengesetzten Seite mit Rasthaken versehen sein, um ein Abgleiten der Konussegmente vom Halteelement effektiver zu verhindern.

Ein Implantationsverfahren, welches nicht Gegenstand der vorliegenden Erfindung ist und lediglich zur Veranschaulichung erläutert wird, kann folgende Schritte umfassen:
a. Adaptieren der Konusgröße der Augmentationsvorrichtung;
b. Einschieben des Applikators in den Kanal der Augmentationsvorrichtung;
c. Einpressen der Augmentationsvorrichtung in den Knochenkanal mittels des Applikators;
d. Entfernen des Applikators aus dem Kanal.

Das Adaptieren der Konusgröße der Augmentationsvorrichtung kann durch Entfernen und/oder Hinzufügen von einem oder mehreren Konussegmenten am proximalen und/oder distalen Konusende erfolgen. Dies ermöglicht sowohl die Anpassung des maximalen und minimalen Konusaußendurchmessers als auch die axiale Erstreckung des Konus.

Die Augmentationsvorrichtung kann bereits eine passende Konusgröße aufweisen, so dass keine Anpassung notwendig ist.

so dass keine Anpassung notwendig ist und Schritt a. übersprungen werden kann.

Um die Augmentationsvorrichtung in einen Patienten zu implantieren, können Augmentationsvorrichtung und Applikator reversibel miteinander verbunden werden. Dies kann durch Einschieben des Applikators in den Kanal der Augmentationsvorrichtung geschehen, so dass das Halteelement des Applikators mit der Konusinnenfläche in Kontakt kommt. Das Anpassen der Konusgröße, falls notwendig, kann vor oder nach dem Verbinden von Augmentationsvorrichtung und Applikator erfolgen.

Zum Einbringen der Augmentationsvorrichtung in den Patienten, insbesondere in den Knochenkanal des Patienten, kann die Augmentationsvorrichtung an der gewünschten Stelle angeordnet werden, gefolgt von einem Krafteintrag auf die Augmentationsvorrichtung über den Applikator. Der Krafteintrag presst die Augmentationsvorrichtung in den Knochenkanal. Insbesondere wird die Augmentationsvorrichtung mit dem distalen Konusende voran in den Knochenkanal eingebracht.

Ist die Augmentationsvorrichtung an der gewünschten Stelle implantiert, kann der Applikator aus dem Kanal, insbesondere mittels einer Zugbewegung, entfernt werden.

Falls gewünscht, kann nach dem Einbringen die Konusgröße durch Hinzufügen oder Entfernen eines Konussegments, insbesondere am proximalen Konusende, variiert werden. Beispielsweise kann ein nach dem Einbringen in den Knochenkanal überstehendes Konussegment entfernt werden.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer Augmentationsvorrichtung,
- Fig. 2: den schematischen Längsschnitt der Augmentationsvorrichtung aus Figur 1 in einer perspektiven Seitenansicht,
- Fig. 3: die Augmentationsvorrichtung aus den Figuren 1 und 2 in einer perspektivischen Seitenansicht,
- Fig. 4: einen schematischen Längsschnitt einer zweiten Augmentationsvorrichtung,
- Fig. 5: den schematischen Längsschnitt der zweiten Augmentationsvorrichtung aus Figur 4 in einer perspektivischen Seitenansicht,
- Fig. 6: einen schematischen Längsschnitt eines Augmentationssystems umfassend die zweite Augmentationsvorrichtung aus den Figuren 4 und 5 und einen Applikator,
- Fig. 7: den schematischen Längsschnitt des Augmentationssystem aus Figur 6 in einer perspektiven Seitenansicht,
- Fig. 8: den Applikator aus den Figuren 6 und 7 in einer perspektivischen Seitenansicht, und
- Fig. 9: ein Flussdiagramm eines Implantationsverfahren, welches nicht Gegenstand der vorliegenden Erfindung ist.

### Beschreibung der Figuren

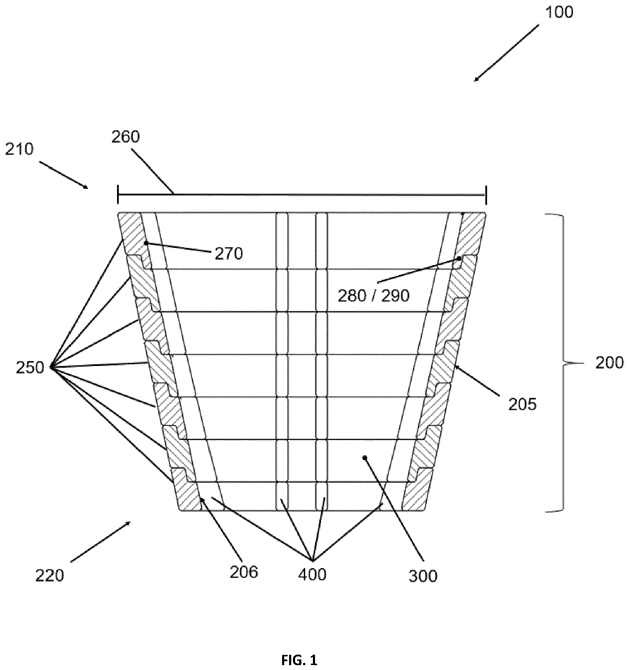

**Figur 1** zeigt einen schematischen Längsschnitt einer beispielhaften Ausführung einer Augmentationsvorrichtung 100. Die Augmentationsvorrichtung 100 umfasst einen ringförmigen Konus 200 mit einem proximalen Konusende 210 und einem axial dem proximalen Konusende 210 entgegengesetztes distalen Konusende 220.

Durch den Konus 200 erstreckt sich vom proximalen Konusende 210 bis zum distalen Konusende 220 ein Kanal 300.

Der Konus 200 selbst ist aus einer Vielzahl, hier sieben, miteinander verbundenen ringförmigen Konussegmenten 250 aufgebaut. Die Konussegmente 250 weisen unterschiedlich große Konussegmentaußendurchmesser 260 auf (lediglich eingezeichnet für das Konussegment 250 am proximalen Konusende 210), wobei das Konussegment am proximalen Konusende 210 den größten Konussegmentaußendurchmesser 260 aufweist und der Konussegmentaußendurchmesser 260 der distal dazu angeordneten Konussegmente 250 bis hin zum distalen Konusende 220 linear abnimmt, so dass eine Konusaußenfläche 205 im Wesentlichen, bis auf die Übergänge von einem Konussegment 250 zu einem dazu benachbarten Konussegment 250, stufenfrei ausgeformt ist. Das Konussegment 250 am distalen Konusende 220 weist den kleinsten Konussegmentaußendurchmesser 260 auf. Die einzelnen Konussegmente 250 weisen eine im Wesentlichen stufenfreie Konussegmentinnenfläche 270 auf (lediglich eingezeichnet für das Konussegment am proximalen Konusende 210), so dass der aus den Konussegmenten 250 zusammengesetzte Konus 200 eine im Wesentlichen glatte Konusinnenfläche 206 aufweist.

Die Konussegmente 250 weisen jeweils eine im Wesentlichen gleiche Wandstärke auf, so dass der Kanal 300, ebenso wie der Konus 200, am proximalen Konusende 210 seine größte Ausdehnung aufweist und die Ausdehnung in Richtung des distalen Konusende immer weiter abnimmt. In einer weiteren, nicht gezeigten Ausführungsform weist der Kanal über die gesamte axiale Erstreckung des Konus 200 die gleiche Ausdehnung auf. In dieser, nicht gezeigten Ausführungsform weisen die einzelnen Konussegmente 250 unterschiedliche Wandstärken auf, wobei die Wandstärke am proximalen Konusende 210 am größten ist und in Richtung des distalen Konusende 220 immer weiter abnimmt.

Bis auf das Konussegment 250 am distalen Konusende 220 weisen alle anderen der in dieser Ausführungsform gezeigten Konussegmente 250 an der dem Kanal 300 zugewandten Konussegmentinnenfläche 270 jeweils einen umlaufenden Steg 290 auf, welcher jeweils mit einer Ausnehmung 280 des jeweils distal benachbarten Konussegments 250 form- und/oder kraftschlüssig zusammenwirkt, um die jeweils benachbarten Konussegmente 250 trennbar voneinander zu verbinden (lediglich eingezeichnet für das Konussegment 250 am proximalen Konusende 210 und das distal dazu benachbarte Konussegment 250). Bis auf das Konussegment 250 am proximalen Konusende 210 weisen alle anderen der in dieser Ausführungsform gezeigten Konussegmente 250 an der dem Kanal 300 zugewandten Konussegmentinnenfläche 270 jeweils eine umlaufende Ausnehmung 280 auf, welche jeweils mit den Stegen 290 der proximal benachbarten Konussegmente 250 wechselwirkt, um die jeweils benachbarten Konussegmente 250 derart miteinander zu verbinden, dass diese trennbar voneinander ausgestaltet sind. Das Konussegment 250 am proximalen Konusende 210 weist in der gezeigten Ausführungsform lediglich einen Steg 280 und das Konussegment 250 am distalen Konusende 220 weist in der gezeigten Ausführungsform lediglich eine Ausnehmung an der jeweiligen Konussegmentinnenfläche 270 auf. Die Konussegmente distal zu dem proximalen Konussegment 250 und proximal zu dem distalen Konussegment 250 weisen an den jeweiligen Konussegmentinnenflächen 270 sowohl eine Ausnehmung 280 als auch einen Steg 290 auf.

In der gezeigten Ausführungsform der Augmentationsvorrichtung 100 weisen alle Konussegmente 250 eine Vielzahl an Führungen 400 auf (nur eingezeichnet für das distale Konussegment 250), um die Augmentationsvorrichtung 100 mit einem Applikator (nicht gezeigt in Figur 1) reversibel zu verbinden. Jedes Konussegment 250 weist vier Führungen 400 auf, wobei eine der Führungen 400 hinter der Zeichenebene in der Abbildung mittig angeordnet ist, zwei der Führungen 400 in der Zeichenebene, und damit nur zur Hälfte, abgebildet sind und eine der Führungen 400 vor der Zeicheneben, und damit nicht abgebildet, ist. Die Führungen 400 sind gleichmäßig über die Konussegmentinnenfläche 270 verteilt. Jede Führung 400 umfasst zwei schienenartige Erhebungen, so dass ein Applikator (nicht gezeigt in Figur 1) mit entsprechenden Gegenstücken zwischen die Erhebungen einer Führung 400 eingreifen kann, um Augmentationsvorrichtung 100 und Applikator zu verbinden. Die einzelnen Führungen 400 der Konussegmente 250 sind dabei derart zueinander ausgerichtet, dass auf der Konusinnenfläche 206 vier schienenartige Konusführungen ausgeformt sind, mit welchen ein Applikator verbindbar ist.

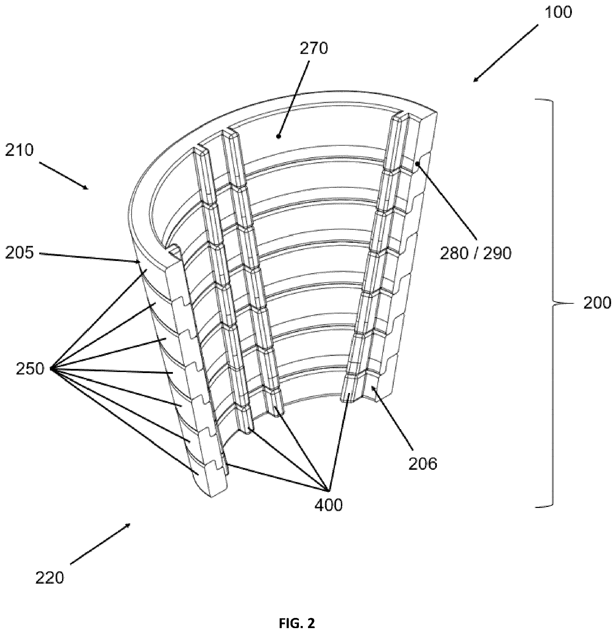

**Figur 2** zeigt den schematischen Längsschnitt der Augmentationsvorrichtung 100 aus Figur 1 in einer perspektivischen Seitenansicht. Zur Vermeidung von Wiederholungen wird auf die vorstehende Beschreibung der Figur 1 verwiesen.

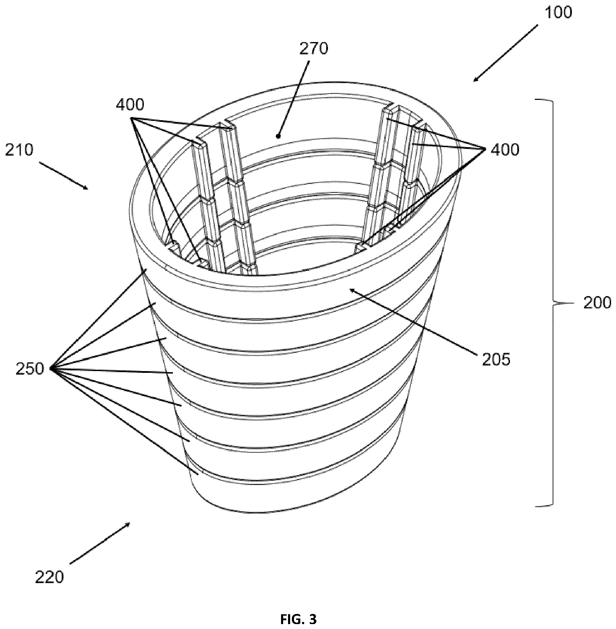

**Figur 3** zeigt die Augmentationsvorrichtung 100 aus den Figuren 1 und 2 in einer perspektivischen Seitenansicht. In der perspektivischen Seitenansicht sind alle vier Führungen 400 sichtbar. Weiterhin ist erkennbar, dass die ringförmigen Konussegmente 250 in einer axialen Draufsicht elliptisch ausgestaltet sind. In der gezeigten Ausführungsform der Augmentationsvorrichtung 100 sind die Konussegmente 250 geschlossen, also ohne Spalt, ausgeformt.

Zur Vermeidung von Wiederholungen wird auf die vorstehende Beschreibung der Figur 1 und 2 verwiesen.

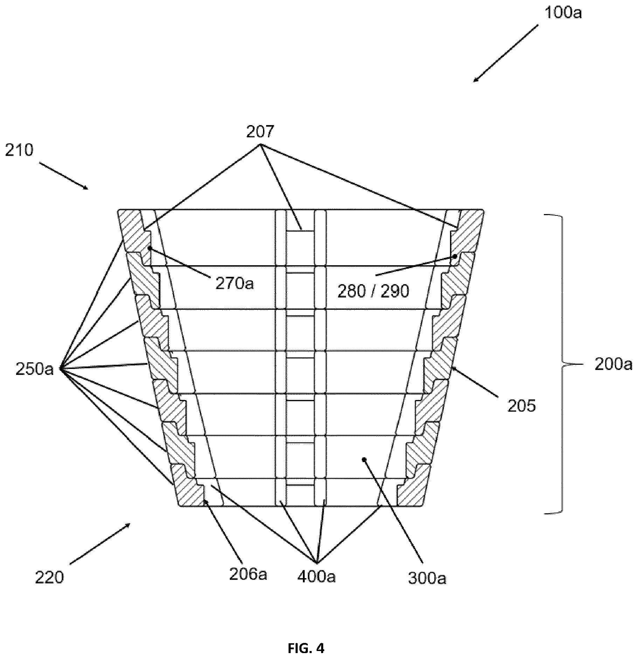

**Figur 4** zeigt eine beispielhafte zweite Ausführungsform einer Augmentationsvorrichtung 100a. Die Ausführungsform gemäß Figur 2 stimmt weitgehend mit der vorstehend beschriebenen und in den Figuren 1 bis 3 dargestellten Ausführungsform überein, so dass zur Vermeidung von Wiederholungen auf die vorstehende Beschreibung der Figuren 1 bis 3 verwiesen wird. Eine Struktur, die aus der Beschreibung der Figuren 1 bis 3 wiederholt wird, weist dasselbe Bezugszeichen auf. Abwandlungen einer gegenüber der in den Figuren 1 bis 3 gezeigten Struktur weisen dasselbe Bezugszeichen mit einem zusätzlichen Buchstaben a auf.

Die Konussegmente 250a der in Figur 4 gezeigten Ausführungsform weisen treppenartige Konuseinkerbungen 207 auf der jeweiligen Konussegmentinnenfläche 270a auf (lediglich eingezeichnet für das Konussegment 250 am proximalen Konusende 210). Die Konuseinkerbungen können mit einem Applikator (nicht eingezeichnet in Figur 4) wechselwirken, um eine bessere Kraftübertragung vom Applikator auf die Augmentationsvorrichtung 100a beim Implantieren in einen Patienten zu gewährleisten. Die Konuseinkerbungen 207 sind im Bereich der Führungen 400a, insbesondere zwischen den zwei Erhebungen einer Führung 400a, angeordnet, während die restliche Konussegmentinnenfläche 270a stufenfrei ausgeformt ist.

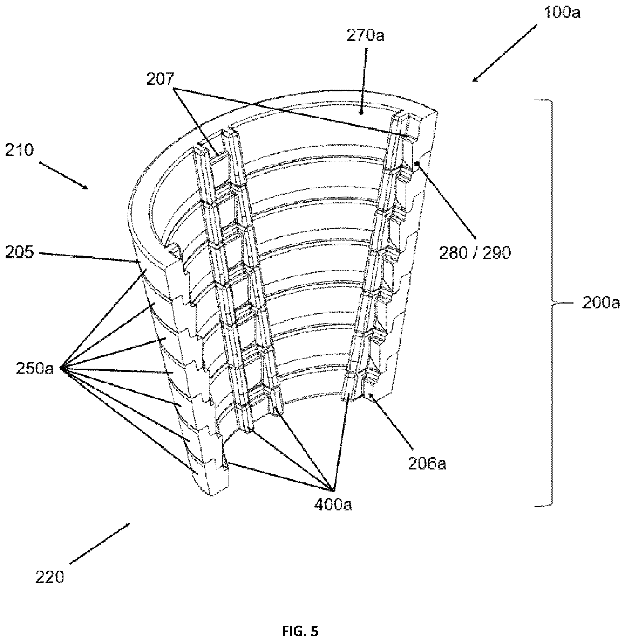

**Figur 5** zeigt den schematischen Längsschnitt der Augmentationsvorrichtung 100a aus Figur 4 in einer perspektivischen Seitenansicht. Zur Vermeidung von Wiederholungen wird auf die vorstehende Beschreibung der Figur 4 verwiesen.

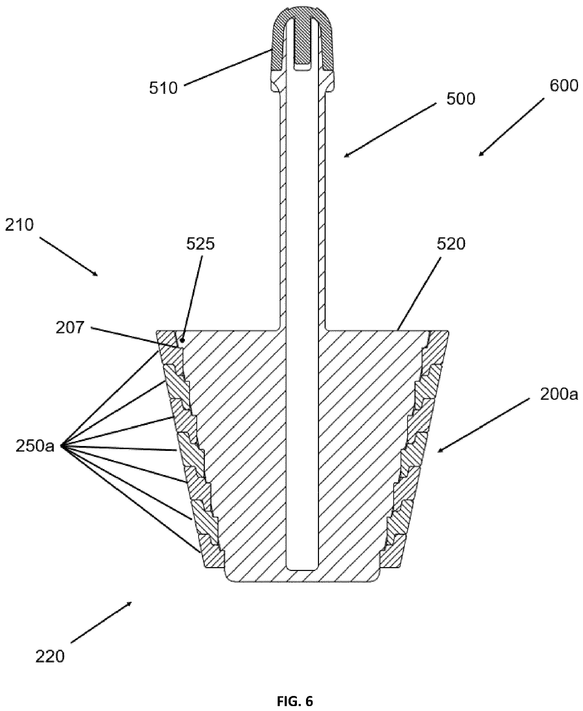

**Figur 6** zeigt einen schematischen Längsschnitt einer beispielhaften Ausführungsform eines Augmentationssystems 600. Das Augmentationssystem 600 umfasst die zweite Ausführungsform der Augmentationsvorrichtung 100a gemäß den Figuren 4 und 5 sowie einen Applikator 500. Zur Vermeidung von Wiederholungen wird bezüglich der Augmentationsvorrichtung 100a auf die vorstehende Beschreibung der Figuren 4 und 5 verwiesen.

Der Applikator 500 umfasst einen Griff 510 um einen Anwender des Augmentationssystems 600 die Handhabung zu erleichtern. Ferner umfasst der Applikator 500 ein mit dem Griff 510 verbundenes Halteelement 520, welches form- und/oder kraftschlüssig mit der Augmentationsvorrichtung 100a verbunden ist. Dazu ist der Applikator 500, insbesondere das Haltelement 520, in den Konus 200a vom proximalen Konusende 210 aus eingeschoben.

In der gezeigten Ausführungsform erstreckt sich das Halteelement 520 über die gesamte axiale Erstreckung des Konus 200a, so dass der Applikator 500 mit jedem einzelnen Konussegment 250a im Bereich der vorbeschriebenen Führungen 400 (siehe Figuren 4 und 5, nicht gezeigt in Figur 6) in Kontakt steht, was eine gute Krafteinleitung auf alle Konussegmente 250a während einer Implantation erlaubt.

Um eine Kraftübertragung vom Applikator 500 auf die Augmentationsvorrichtung 100a beim Implantieren derselben in einen Patienten zu verbessern, weist das Halteelement Halteelementeinkerbungen 525, insbesondere treppenartige Halteelementeinkerbungen 525, auf (lediglich eingezeichnet am dem proximalen Konusende 210 zugewandten Abschnitt des Halteelements 520). Die Haltelementeinkerbungen 525 sind in form- und/oder kraftschlüssigem Eingriff mit den Konuseinkerbungen 207. Dies erlaubt eine gute Krafteinleitung aus Richtung des proximalen Konusende 210 auf alle Konussegmente 250a und ermöglicht gleichzeitig ein einfaches Lösen von Augmentationsvorrichtung 100a und Applikator 500 nach erfolgter Implantation der Augmentationsvorrichtung 100a.

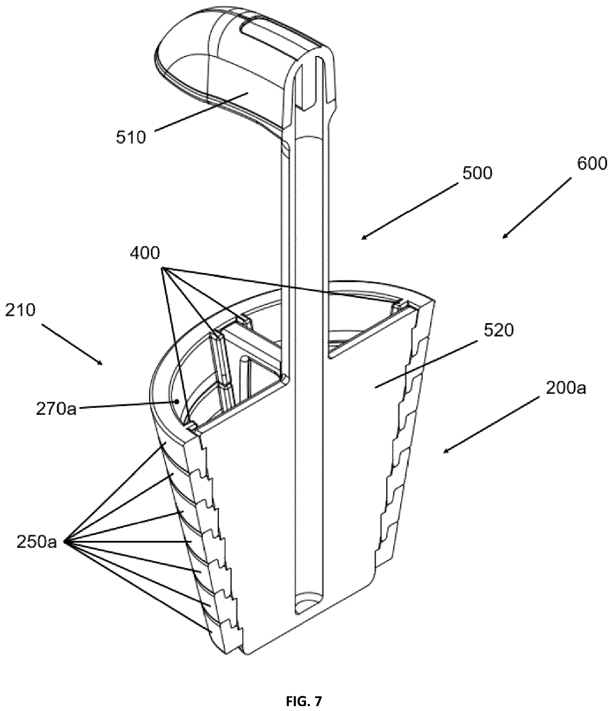

**Figur 7** zeigt den schematischen Längsschnitt des Augmentationssystems 600 aus Figur 6 in einer perspektivischen Seitenansicht. Zur Vermeidung von Wiederholungen wird auf die vorstehende Beschreibung der Figur 6 verwiesen.

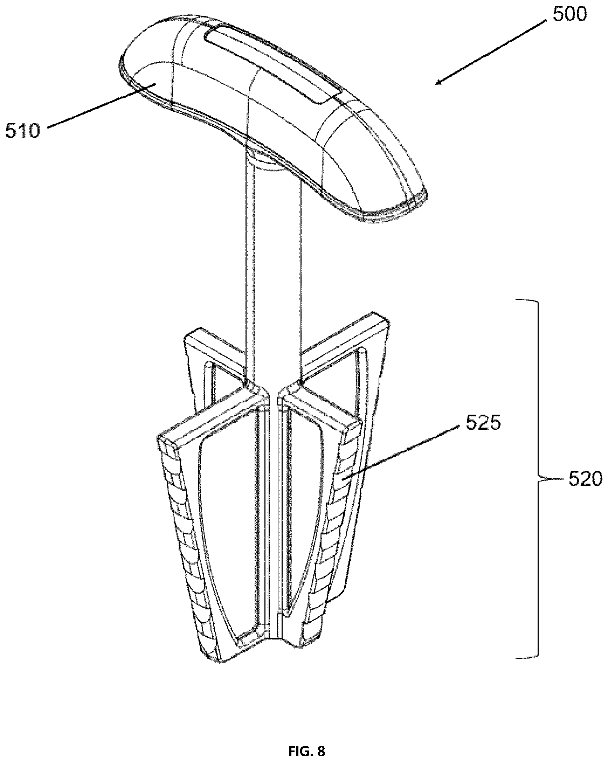

In der perspektivischen Seitenansicht der Figur 7 ist erkennbar, dass das Halteelement 520 vier Ausleger umfasst, welche in die Führungen 400 des Konus 200a eingreifen (im Längsschnitt ist lediglich ein Ausleger vollständig, zwei Ausleger in der Zeicheneben teilweise zu sehen. Der vierte Ausleger ist im abgeschnittenen Teil des Augmentationssystems 600 angeordnet). **Figur 8** zeigt den Applikator 500 aus den Figuren 6 und 7 in einer perspektivischen Seitenansicht. Zur Vermeidung von Wiederholungen wird auf die vorstehende Beschreibung der Figuren 6 und 7 verwiesen.

Der Griff 510 ist auf seiner proximalen, dem Haltelement 520 abgewandten Seite dazu ausgelegt, mit einem Werkzeug, insbesondere einem Hammer, bearbeitet zu werden, um eine Augmentationsvorrichtung 100, 100a in einen Patienten mit Druck zu implantieren.

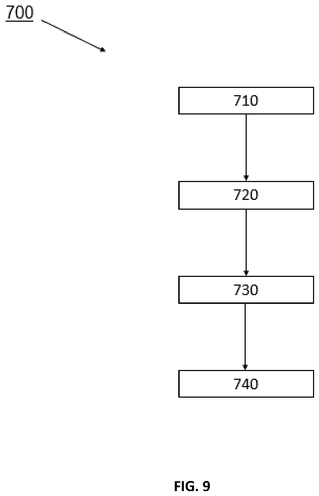

**Figur 9** zeigt ein Flussdiagramm eines Implantationsverfahrens 700, welches nicht Gegenstand der vorliegenden Erfindung ist, unter Verwendung des Augmentationssystems 600 umfassend die Schritte 710 bis 740. Das Augmentationssystem 600 umfasst die Augmentationsvorrichtung 100, 100a und den Applikator 500. Der Applikator 500 umfasst den Griff 510 und das mit dem Griff 510 verbundene Halteelement 520. Das Implantationsverfahren 700 kann einer Implantation der Augmentationsvorrichtung 100, 100a in einen Knochenkanal eines Patienten dienen.

In Schritt 710 erfolgt ein Adaptieren der Konusgröße der Augmentationsvorrichtung 100, 100a an die anatomischen Gegebenheiten des zu behandelnden Patienten. Dies kann durch Hinzufügen und/oder Entfernen von Konussegmenten 250, 250a am proximalen Konusende 210 und/oder am distalen Konusende 220 geschehen. Beispielsweise verringert ein Entfernen des Konussegments 250, 250a mit dem größten Konussegmentaußendurchmesser 260 am proximalen Konusende 210 den maximalen Konusaußendurchmesser und die axiale Erstreckung des Konus 200, 200a.

In Schritt 720 erfolgt ein Einschieben des Applikators 500 in den Kanal 300, 300a der Augmentationsvorrichtung 100, 100a um Applikator 500 und Augmentationsvorrichtung 100, 100a reversibel zu verbinden. Durch das Einschieben kommt das Halteelement 520 des Applikators 500 mit der Konusinnenfläche 206, 206a, bevorzugt mit der Konussegmentinnenfläche 270, 270a an jedem einzelnen Konussegment 250, 250a, in Kontakt, wodurch insbesondere eine form- und/oder kraftschlüssige Verbindung entsteht.

Der Schritt 710 kann dabei vor oder nach dem Schritt 720 durchgeführt werden.

In Schritt 730 erfolgt ein Einpressen der Augmentationsvorrichtung 100, 100a in den Knochenkanal unter Zuhilfenahme des Applikators 500. Dazu hält der Anwender des Augmentationssystems 600 den Applikator 500 am Griff 510 und presst das Augmentationssystem 600 mit dem distalen Konusende 220 voran in den Knochenkanal des Patienten. Dies kann mittels reiner Muskelkraft oder auch unter Verwendung eines Werkzeugs, wie beispielsweise eines Hammers, geschehen. Der Schritt 710, insbesondere ein Hinzufügen oder Entfernen eines Konussegments 250, 250a am proximalen Konusende 210, kann dabei vor oder nach dem Schritt 730 durchgeführt werden.

Ist die Augmentationsvorrichtung 100, 100a in den Knochenkanal eingebracht, wird in Schritt 740 der Applikator 500 aus dem Kanal 300, 300a entfernt und somit der Applikator 500 von der Augmentationsvorrichtung 100, 100a getrennt. Der Schritt 710, insbesondere ein Hinzufügen oder Entfernen eines Konussegments 250, 250a am proximalen Konusende 210, kann dabei vor oder nach dem Schritt 740 durchgeführt werden.

### Bezugszeichen

- 100, 100a: Augmentationsvorrichtung
- 200,200a: Konus
- 205: Konusaußenfläche
- 206, 206a: Konusinnenfläche
- 207: Konuseinkerbung
- 210: proximales Konusende
- 220: distales Konusende
- 300: Kanal
- 250,250a: Konussegment
- 260: Konussegmentaußendurchmesser
- 270,270a: Konussegmentinnenfläche
- 280: Ausnehmung
- 290: Steg
- 400,400a: Führung
- 500: Applikator
- 510: Griff
- 520: Halteelement
- 525: Halteelementeinkerbung
- 600: Augmentationssystem
- 700: Implantationsverfahren
- 710: Adaptieren
- 720: Einschieben
- 730: Einpressen
- 740: Entfernen

## Patentansprüche

1. Augmentationsvorrichtung (100, 100a) umfassend
einen ringförmigen Konus (200, 200a), der einen Kanal (300, 300a) umgibt, welcher sich von einem proximalen Konusende (210) zu einem distalen Konusende (220) des Konus (200, 200a) durch den Konus (200, 200a) erstreckt,
wobei der Konus (200, 200a) aus einer Vielzahl miteinander verbundener, ringförmiger Konussegmente (250, 250a) ausgebildet ist, wobei ein Konussegmentaußendurchmesser (260) der einzelnen Konussegmente (250, 250a) vom proximalen Konusende (210) zum distalen Konusende (220) abnimmt und wobei die Konussegmente (250, 250a) trennbar voneinander ausgestaltet sind, so dass der Konus (200, 200a) eine adaptierbare Konusgröße aufweist,
**dadurch gekennzeichnet, dass**
die Konussegmente (250, 250a) form- und/oder kraftschlüssig miteinander verbunden sind.

2. Augmentationsvorrichtung (100, 100a) nach Anspruch 1, wobei die Konussegmente (250, 250a) reversibel trennbar voneinander ausgestaltet sind.

3. Augmentationsvorrichtung (100, 100a) nach einem der vorhergehenden Ansprüche, wobei die Konussegmente (250, 250a) mittels einer Nut-Feder-Verbindung miteinander verbunden sind.

4. Augmentationsvorrichtung (100, 100a) nach einem der vorhergehenden Ansprüche, wobei die Konussegmente (250, 250a) jeweils eine Ausnehmung (280) und/oder einen Steg (290) aufweisen, wobei die Ausnehmung (280) und der Steg (290) zweier im Konus (200, 200a) benachbarter Konussegmente (250, 250a) zusammenwirken, um die zwei im Konus (200, 200a) benachbarten Konussegmente (250, 250a) miteinander zu verbinden.

5. Augmentationsvorrichtung (100, 100a) nach Anspruch 4, wobei die Ausnehmung (280) und/oder der Steg (290) an einer Konussegmentinnenfläche (270, 270a) angeordnet sind/ist.

6. Augmentationsvorrichtung (100, 100a) nach einem der vorhergehenden Ansprüche, wobei der Konus (200, 200a) eine dem Kanal (300, 300a) abgewandte, stufenfreie Konusaußenfläche (205) aufweist.

7. Augmentationsvorrichtung (100, 100a) nach einem der vorhergehenden Ansprüche, wobei die Konussegmente (250, 250a) jeweils eine Führung (400, 400a) aufweisen, um den Konus (200, 200a) mit einem Applikator (500) reversibel zu verbinden.

8. Augmentationsvorrichtung (100, 100a) nach Anspruch 7, wobei die Führung (400, 400a) dem Kanal (300, 300a) zugewandt ist, so dass der Applikator (500) durch Einbringen in den Kanal (300, 300a) mit dem Konus (200, 200a) reversibel verbindbar ist.

9. Augmentationsvorrichtung (100, 100a) nach einem der vorhergehenden Ansprüche, wobei die Konussegmente (250, 250a) jeweils in Form eines geschlossenen Rings ausgebildet sind.

10. Augmentationsvorrichtung (100, 100a) nach einem der vorhergehenden Ansprüche, wobei die Konussegmente (250, 250a) ein Polymer, ein Metall und/oder eine Metalllegierung umfassen.

11. Augmentationssystem (600) umfassend eine Augmentationsvorrichtung (100, 100a) nach einem der Ansprüche 1 bis 10 und einen Applikator (500), der Applikator (500) umfassend einen Griff (510) und ein mit dem Griff (510) verbundenes Halteelement (520), wobei das Halteelement (520) derart ausgeformt ist, dass bei einem Einschieben des Applikators (500) in den Kanal (300, 300a) das Haltelement (520) an einer Konusinnenfläche (206, 206a) anliegt und so die Augmentationsvorrichtung (100, 100a) und den Applikator (500) reversibel miteinander verbindet.

12. Augmentationssystem (600) nach Anspruch 11, wobei der Applikator (500) in den Kanal (300, 300a) vom proximalen Konusende (210) aus einschiebbar ist.

13. Augmentationssystem (600) nach Anspruch 11 oder 12, wobei an dem Halteelement (520) Halteelementeinkerbungen (525) ausgebildet sind, welche mit Konuseinkerbungen (207) an der Konusinnenfläche (206, 206a) in Eingriff gebracht werden können.

## Claims

1. An augmentation device (100, 100a) comprising
an annular cone (200, 200a), which surrounds a channel (300, 300a) extending through the cone (200, 200a) from a proximal cone end (210) to a distal cone end (220) of the cone (200, 200a),
the cone (200, 200a) being formed by a plurality of interconnected annular cone segments (250, 250a), a cone segment outer diameter (260) of the individual cone segments (250, 250a) decreasing from the proximal cone end (210) to the distal cone end (220) and the cone segments (250, 250a) being separable from one another so that the cone (200, 200a) has an adaptable cone size,
**characterized in that**
the cone segments (250, 250a) are interconnected in an interlocking and/or frictional manner.

2. The augmentation device (100, 100a) according to claim 1, wherein the cone segments (250, 250a) are designed to be reversibly separable from one another.

3. The augmentation device (100, 100a) according to either of the preceding claims, wherein the cone segments (250, 250a) are interconnected by means of a tongue and groove connection.

4. The augmentation device (100, 100a) according to any of the preceding claims, wherein the cone segments (250, 250a) each have a recess (280) and/or a projection (290), wherein the recess (280) and the projection (290) of two cone segments (250, 250a) adjacent in the cone (200, 200a) interact in order to interconnect the two cone segments (250, 250a) adjacent in the cone (200, 200a).

5. The augmentation device (100, 100a) according to claim 4, wherein the recess (280) and/or the projection (290) are arranged on a cone segment inner surface (270, 270a).

6. The augmentation device (100, 100a) according to any of the preceding claims, wherein the cone (200, 200a) has a step-free cone outer surface (205) facing away from the channel (300, 300a).

7. The augmentation device (100, 100a) according to any of the preceding claims, wherein the cone segments (250, 250a) each have a guide (400, 400a) for reversibly connecting the cone (200, 200a) to an applicator (500).

8. The augmentation device (100, 100a) according to claim 7, wherein the guide (400, 400a) faces the channel (300, 300a) such that the applicator (500) is reversibly connectable to the cone (200, 200a) by being inserted into the channel (300, 300a).

9. The augmentation device (100, 100a) according to any of the preceding claims, wherein the cone segments (250, 250a) are each in the form of a closed ring.

10. The augmentation device (100, 100a) according to any of the preceding claims, wherein the cone segments (250, 250a) comprise a polymer, a metal and/or a metal alloy.

11. An augmentation system (600) comprising an augmentation device (100, 100a) according to any of claims 1 to 10 and an applicator (500), the applicator (500) comprising a handle (510) and a holding element (520) connected to the handle (510), wherein the holding element (520) is shaped such that, upon insertion of the applicator (500) into the channel (300, 300a), the holding element (520) abuts against a cone inner surface (206, 206a) and thus reversibly interconnects the augmentation device (100, 100a) and the applicator (500).

12. The augmentation system (600) according to claim 11, wherein the applicator (500) is insertable into the channel (300, 300a) from the proximal cone end (210).

13. The augmentation system (600) according to claim 11 or 12, wherein holding element notches (525) are formed in the holding element (520), which notches can be brought into engagement with cone notches (207) on the cone inner surface (206, 206a).

## Revendications

1. Dispositif d'augmentation (100, 100a) comprenant
un cône (200, 200a) annulaire qui entoure un canal (300, 300a), lequel canal s'étend à travers le cône (200, 200a) d'une extrémité proximale de cône (210) à une extrémité distale de cône (220) du cône (200, 200a),
dans lequel le cône (200, 200a) est formé d'une pluralité de segments de cône (250, 250a) annulaires reliés les uns aux autres, dans lequel un diamètre externe de segment de cône (260) des segments de cône (250, 250a) individuels diminue de l'extrémité proximale de cône (210) à l'extrémité distale de cône (220) et dans lequel les segments de cône (250, 250a) sont conçus de manière à pouvoir être séparés les uns des autres, de sorte que le cône (200, 200a) présente une taille de cône adaptable,
**caractérisé en ce que**
les segments de cône (250, 250a) sont reliés les uns aux autres par complémentarité de formes et/ou à force.

2. Dispositif d'augmentation (100, 100a) selon la revendication 1, dans lequel les segments de cône (250, 250a) sont conçus de manière à pouvoir être séparés les uns des autres de manière réversible.

3. Dispositif d'augmentation (100, 100a) selon l'une des revendications précédentes, dans lequel les segments de cône (250, 250a) sont reliés les uns aux autres au moyen d'une liaison à rainure et languette.

4. Dispositif d'augmentation (100, 100a) selon l'une des revendications précédentes, dans lequel les segments de cône (250, 250a) présentent respectivement un évidement (280) et/ou une traverse (290), dans lequel l'évidement (280) et la traverse (290) de deux segments de cône (250, 250a) voisins dans le cône (200, 200a) coopèrent pour relier l'un à l'autre les deux segments de cône (250, 250a) voisins dans le cône (200, 200a).

5. Dispositif d'augmentation (100, 100a) selon la revendication 4, dans lequel l'évidement (280) et/ou la traverse (290) sont disposés sur une surface interne de segment de cône (270, 270a).

6. Dispositif d'augmentation (100, 100a) selon l'une des revendications précédentes, dans lequel le cône (200, 200a) présente une surface externe de cône (205) continue opposée au canal (300, 300a).

7. Dispositif d'augmentation (100, 100a) selon l'une des revendications précédentes, dans lequel les segments de cône (250, 250a) présentent respectivement un guide (400, 400a) pour relier le cône (200, 200a) à un applicateur (500) de manière réversible.

8. Dispositif d'augmentation (100, 100a) selon la revendication 7, dans lequel le guide (400, 400a) fait face au canal (300, 300a), de sorte que l'applicateur (500) peut être relié de manière réversible au cône (200, 200a) par introduction dans le canal (300, 300a).

9. Dispositif d'augmentation (100, 100a) selon l'une des revendications précédentes, dans lequel les segments de cône (250, 250a) sont formés respectivement sous la forme d'une bague fermée.

10. Dispositif d'augmentation (100, 100a) selon l'une des revendications précédentes, dans lequel les segments de cône (250, 250a) comprennent un polymère, un métal et/ou un alliage métallique.

11. Système d'augmentation (600) comprenant un dispositif d'augmentation (100, 100a) selon l'une des revendications 1 à 10 et un applicateur (500), l'applicateur (500) comprenant une poignée (510) et un élément de retenue (520) relié à la poignée (510), dans lequel l'élément de retenue (520) est façonné de telle sorte que, lors d'une insertion de l'applicateur (500) dans le canal (300, 300a), l'élément de retenue (520) repose sur une surface interne de cône (206, 206a) et relie ainsi le dispositif d'augmentation (100, 100a) et l'applicateur (500) l'un à l'autre de manière réversible.

12. Système d'augmentation (600) selon la revendication 11, dans lequel l'applicateur (500) peut être inséré dans le canal (300, 300a) à partir de l'extrémité proximale de cône (210).

13. Système d'augmentation (600) selon la revendication 11 ou 12, dans lequel des encoches d'élément de retenue (525) sont formées sur l'élément de retenue (520), lesquelles peuvent être mises en prise avec des encoches de cône (207) sur la surface interne de cône (206, 206a).
